(19) Europäisches Patentamt
European Patent Office
Office européen des brevets

(11) Veröffentlichungsnummer: **0 476 554 A1**

(12) **EUROPÄISCHE PATENTANMELDUNG**

(21) Anmeldenummer: **91115630.5**

(51) Int. Cl.5: **C07D 239/52**, C07D 239/58

(22) Anmeldetag: **14.09.91**

(30) Priorität: **15.09.90 EP 90117823**

(43) Veröffentlichungstag der Anmeldung:
**25.03.92 Patentblatt 92/13**

(84) Benannte Vertragsstaaten:
**BE CH DE DK ES FR GB GR IT LI NL**

(71) Anmelder: **HOECHST AKTIENGESELLSCHAFT**
**Postfach 80 03 20**
**W-6230 Frankfurt am Main 80(DE)**

(72) Erfinder: **Lachhein, Stephen, Dr.**
**Kiedricher Strasse 18**
**W-6238 Hofheim am Taunus(DE)**
Erfinder: **Mildenberger, Hilmar, Dr.**
**Fasanenstrasse 24**
**W-6233 Kelkheim/Ts(DE)**

(54) **Verfahren zur Herstellung von Aminopyridinen.**

(57) 2-Aminopyrimidine der Formel I

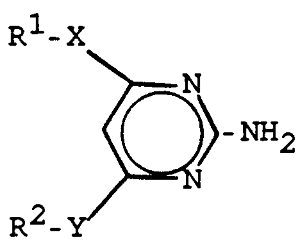

(I),

worin
X ,Y = O oder S
$R^1$, $R^2$ = unabhängig voneinander niederes Alkyl, Alkoxyalkyl oder Haloalkyl
bedeuten, werden durch Umsetzung von Propandiimidaten der Formel II

$R^1$-X-C(=NH)-$CH_2$-C(=NH)-Y-$R^2$ (II)

oder deren Salzen mit Cyanamid in Gegenwart einer Base bei pH-Werten oberhalb von pH 7 erhalten.

Das Verfahren ermöglicht die Herstellung der Aminopyrimidine in einer Eintopfreaktion.

EP 0 476 554 A1

Gegenstand der der Erfindung ist ein Verfahren zur Herstellung von Pyrimidinen der Formel I

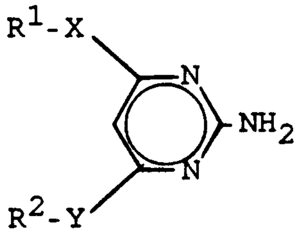

(I),

worin

X und Y jeweils Sauerstoff oder Schwefel und

$R^1$, $R^2$ = unabhängig voneinander ($C_1$-$C_4$)-Alkyl, ($C_1$-$C_4$)-Alkoxy-($C_1$-$C_2$)alkyl oder Halo($C_1$-$C_4$)alkyl

bedeuten,

durch Umsetzung eines Propandiimidats der Formel II

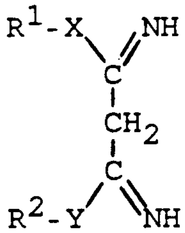

(II)

oder dessen Salz mit Cyanamid ($NH_2$-CN) in Gegenwart einer Base, dadurch gekennzeichnet, daß man das Propandiimidat oder dessen Salz bei pH-Werten oberhalb von pH 7 bis pH 14 zu einer Lösung einer Base und Cyanamid in einem inerten Lösungsmittel gibt und umsetzt.

Verbindungen der Formel I sind wertvolle Zwischenprodukte bei der Herstellung von Sulfonylharnstoffen mit herbizider Wirkung (US-PS 4 169 179, EP-A 071 958).

Bekannt ist die Herstellung der Pyrimidine der Formel I durch Umsetzung der Propandiimidate mit wäßriger Cyanamidlösung (EP-A 0 024 200).

Dieses Verfahren ist gekennzeichnet durch eine mehrstufige Reaktionsfolge mit

1) Umsetzung der Verbindung der Formel II mit Basen,
2) Umsetzung mit Cyanamid unter Bildung eines isolierbaren Zwischenprodukts und
3) Cyclisierung zu Verbindungen der Formel I.

Besonder nachteilig ist dabei die Notwendigkeit der Reaktionsführung in einem engen pH-Bereich (pH-Wert 5-7). Neben verfahrenstechnischen Problemen treten in der sauren wäßrigen Lösung Hydrolysereaktionen des als Ausgangsstoff dienenden Propandiimidates, bzw. dessen Di- oder Monosalzes, auf (R. Rogers and D.G. Neilson, Chem. Reviews, 61, S. 179, 1961). Dadurch werden Nebenprodukte in nicht unerheblichem Maß gebildet, was bei den Endprodukten zu unbefriedigenden Reinheiten und Ausbeuten führt.

Überraschenderweise stellt das erfindungsgemäße Verfahren demgegenüber einen verfahrenstechnisch einfachen Ein- oder Zweistufenprozeß ohne isolierbare Zwischenprodukte dar, der eine Reaktionsführung ohne spezielle pH-Kontrolle als Eintopfverfahren gestattet. Nebenprodukte werden nur in untergeordnetem Maß gebildet.

Eine solche Erhöhung der Ausbeuten und Reinheiten der Endprodukte war aufgrund der Erhöhung des pH-Wertes auf Werte größer 7 und des direkten Kontaktes aller Ausgangsstoffe (Propandiimidat, Cyanamid und Base) nicht zu erwarten.

Das erfindungsgemäße Verfahren wir zweckmäßig so durchgeführt, daß man das Propandiimidat als Salz, z. B als Dihydrochloridsalz, zu einer Lösung von Base und Cyanamid in einem inerten Lösungsmittel bei Reaktionstemperaturen von -20 bis 200 °C, vorzugsweise -10 bis 150 °C und einem pH-Wert oberhalb von 7, vorzugsweise 7,1 bis 12, insbesondere 8 bis 11 gibt und umsetzt. Die Lösung von Base und Cyanamid enthält vorzugsweise mindestens 1 Äquivalent an Base und mindestens 1 Mol an Cyanamid pro Mol eingesetzem Propandiimidat. Insbesondere ist ein geringer, z. B. bis 10%iger Basenüberschuß bevorzugt. Dadurch wird sichergestellt, daß während der gesamten Reaktionsdauer der pH-Wert größer als

7 ist.

Als Salze des Propandiimidates werden bevorzugt solche der Fluor-, Chlor- oder Bromwasserstoffsäure, der Schwefel- oder Phosphorsäure eingesetzt. Haloalkylreste für $R^1$, $R^2$ sind beispielsweise $CH_2CH_2Cl$ oder $CH_2CF_3$. Bevorzugt bedeuten X und Y jeweils Sauerstoff; $R^1$, $R^2$ sind vorzugsweise jeweils $(C_1$-$C_4)$Alkyl, insbesondere Methyl.

Als inerte Lösungsmittel können Wasser, Alkohole wie Methanol, Ethanol und Propanol; Ketone wie Aceton und Methylisobutylketon; Ether wie Diethylether, Dioxan und Tetrahydrofuran; Ester wie Methylacetat, Ethylacetat und Butylacetat; Kohlenwasserstoffe wie Toluol, Xylol und Cyclohexan; Nitrile wie Acetonitril und halogenierte Kohlenwasserstoffe wie Dichlormethan oder Chloroform oder deren Mischungen verwendet werden.

Als Basen können z.B. Alkali- und Erdalkalihydroxide, -carbonate, -hydrogencarbonate oder -alkoholate verwendet werden.

Um störende Einflüsse von Sauerstoff auf die Reaktion zu vermeiden, ist es vorteilhaft unter Inertgasatmosphäre, beispielsweise unter Stickstoff, zu arbeiten.

Die Verbindungen der Formel II können nach bekannten Methoden hergestellt werden (S.M. McElvain and I.D. Schroeder, J. Am. Chem. Soc. 71, 40 (1949); B. Harstun, DE-A 2 426 913).

Nachfolgende Beispiele sollen das erfindungsgemäße Verfahren näher erläutern:

**Beispiel 1**

Zu einer Lösung von 50 g Kaliumhydrogencarbonat, 250 ml Wasser und 27,3 g Cyanamid mit einem eingestellten pH-Wert von 8,5 werden unter Stickstoffüberlagerung bei 0 °C 101,5 g Dimethyl-propandiimidat-dihydrochlorid gegeben. Nach einer Nachrührzeit von 4 Stunden wird vom ausgefallenen Feststoff abgesaugt und in 400 ml Toluol am Wasserabscheider erhitzt. Die erhaltene Lösung wird heiß filtriert und das Lösungsmittel im Vakuum entfernt.
Es verbleiben 69,7 g Produkt, 2-Amino-4,6-dimethoxypyrimidin, von einer Reinheit von 99,4 %, was einer Ausbeute von 90 % d.Th. entspricht. Der Schmelzpunkt beträgt 93-95 °C.

**Beispiel 2**

25 g Kaliumhydrogencarbonat, 75 ml Wasser, 13,6 g Cyanamid und 200 ml Methylisobutylketon werden zusammengegeben und auf 0 °C abgekühlt. Es stellt sich ein pH-Wert von 8,5 ein. Zu dieser Lösung werden 50,8 g Dimethyl-propandiimidat-dihydrochlorid gegeben und für 4 Stunden nachgerührt. Nach Erhitzen der Lösung auf 100 °C wird heiß filtriert und für weitere 2 Stunden am Rückfluß erhitzt. Nach Entfernen des Lösungsmittels im Vakuum werden 34,7 g 2-Amino-4,6-dimethoxypyrimidin von einer Reinheit von 98,2 % erhalten, was einer Ausbeute von 89,8 % d.Th. entspricht.

**Beispiel 3**

27 g Natriummethylat in 250 ml Methanol und 21 g Cyanamid werden bei einem PH-Wert oberhalb von 7 und einer Temperatur von 0 °C mit 101,5 g Dimethyl-propandiimidat-dihydrochlorid versetzt. Nach 4 Stunden Rühren bei Raumtemperatur wird vom Salz abfiltriert und das Lösungsmittel mit Xylol abdestilliert. Es verbleiben 68,9 g 2-Amino-4,6-dimethoxypyrimidin von einer Reinheit von 98,4 %, was einer Ausbeute von 89,4 % d.Th. entspricht.

**Beispiel 4 (ohne Stickstoff als Schutzgas)**

Zu einer Lösung von 50 g Kaliumhydrogencarbonat, 312,5 ml Wasser und 27,3 g Cyanamid mit einem eingestellten pH-Wert von 8,5 werden bei 0°C 101,5 g Dimethyl-propandiimidat-dihydrochlorid gegeben. Nach einer Nachrührzeit von 4 Stunden wird von ausgefallenen Feststoff abgesaugt und in 400 ml Toluol am Wasserabscheider erhitzt.
Die erhaltene Lösung wird heiß filtriert und das Lösungsmittel im Vakuum entfernt.
Es verbleiben 69,4 g Produkt; 2-Amino-4,6-dimethoxypyrimidin, von einer Reinheit von 99,1 % was einer Ausbeute von 89,3 % d.Th. entspricht. Der Schmelzpunkt beträgt 93 - 95°C.

**Vergleichsbeispiel 1** (nach EP-A 0 024 200)

62 g Natriumhydrogencarbonat werden in 600 ml Wasser gelöst und mit 50 g Dimethyl-propandiimidat-dihydrochlorid bei 0 °C und einem pH-Wert von 5 bis 7 versetzt. Nach vollständiger Lösung werden 65 g einer 50%igen Cyanamidlösung zugegeben. Nach 2 Stunden Rühren bei Raumtemperatur wird der Niederschlag abgesaugt und die wäßrige Phase einmal mit Methylenchlorid extrahiert und das Lösungsmittel im Vakuum entfernt. Es wurden 82,4 g 3-Amino-3-methoxy-N-cyano-2-propenimidat erhalten, die mit 1000 ml Toluol am Rückfluß für 2 Stunden erhitzt werden. Nach Entfernen des Lösungsmittels verbleiben 79,8 g Produkt von einer Reinheit von 95,8 %, was einer Ausbeute von 71,3 % d. Theorie entspricht. Der Schmelzpunkt ist 92 - 94 °C.

**Vergleichsbeispiel 2** (nach EP-A 0 024 200)

62 g Natriumhydrogencarbonat werden in 600 ml Wasser gelöst und mit 150 g Dimethyl-propandiimidat-dihydrochlorid bei 0 °C und einem pH-Wert von 5-7 versetzt. Nach vollständiger Lösung werden 92 g einer 50 %igen Cyanamidlösung zugegeben. Nach 3 Stunden Rühren wird abgesaugt und mit 1000 ml Toluol für 2 Stunden zum Rückfluß erhitzt. Nach Entfernen des Lösungsmittels verbleiben 76,0 g Produkt von einer Reinheit von 94,8 %, was einer Ausbeute von 67,2 % d.Th. entspricht. Der Schmelzpunkt ist 92-94 °C.

Analog zu den in den Beispielen 1-3 beschriebenen Verfahrensweisen lassen sich beispielsweise folgende Verbindungen der Formel I herstellen:

| Beispiel | X | Y | $R^1$ | $R^2$ |
|---|---|---|---|---|
| 4 | O | O | $C_2H_5$ | $C_2H_5$ |
| 5 | O | O | $CH-CH_3$<br>$CH_3$ | $CH-CH_3$<br>$CH_3$ |
| 6 | S | S | $CH_3$ | $CH_3$ |
| 7 | S | S | $C_2H_5$ | $C_2H_5$ |
| 8 | S | S | $CH-CH_3$<br>$CH_3$ | $CH-CH_3$<br>$CH_3$ |
| 9 | O | O | $CH_2-CH_2-OCH_3$ | $CH_2-CH_2-OCH_3$ |
| 10 | O | O | $CF_2H$ | $CF_2H$ |
| 11 | O | O | $CH_2CF_3$ | $CH_2CF_3$ |
| 12 | O | O | $CH_2FCF_2H$ | $CH_2FCF_2H$ |
| 13 | O | O | $CH_2CH_2Cl$ | $CH_2CH_2Cl$ |

**Patentansprüche**

1. Verfahren zur Herstellung von Verbindungen der Formel I

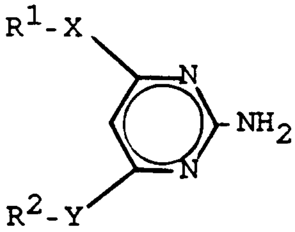

(I),

worin

X und Y jeweils Sauerstoff oder Schwefel und

$R^1$, $R^2$ unabhängig voneinander $(C_1-C_4)$-Alkyl, $(C_1-C_4)$-Alkoxy-$(C_1-C_2)$alkyl oder Halo$(C_1-C_4)$-alkyl

bedeuten,

durch Umsetzung eines Propandiimidats der Formel II

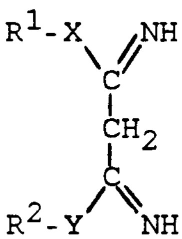

(II)

oder dessen Salzes mit Cyanamid ($NH_2$-CN) in Gegenwart einer Base, dadurch gekennzeichnet, daß man das Propandiimidat oder dessen Salz bei pH-Werten oberhalb von pH 7 bis pH 14 zu einer Lösung einer Base und Cyanamid in einem inerten Lösungsmittel gibt und umsetzt.

2. Verfahren gemäß Anspruch 1, dadurch gekennzeichnet, daß man die Reaktion bei pH-Werten von 7,1 - 12 durchführt.

3. Verfahren nach Anspruch 2, dadurch gekennzeichnet, daß der pH-Wert 8 bis 11 ist.

4. Verfahren gemäß Anspruch 1, 2 oder 3, dadurch gekennzeichnet, daß in Formel I X und Y jeweils ein Sauerstoffatom und $R^1$ und $R^2$ jeweils $(C_1-C_4)$Alkyl bedeuten.

5. Verfahren nach Anspruch 4, dadurch gekennzeichnet, daß $R^1$ und $R^2$ jeweils Methyl bedeuten.

6. Verfahren gemäß einem oder mehreren der Ansprüche 1 bis 5, dadurch gekennzeichnet, daß man als inertes Lösungsmittel Wasser, einen Alkohol, ein Keton, einen Kohlenwasserstoff, einen halogenierten Kohlenwasserstoff, einen Ether, einen Ester, ein Nitril oder deren Mischungen verwendet.

7. Verfahren gemäß einem oder mehreren der Ansprüche 1 bis 6, dadurch gekennzeichnet, daß man als Salze der Verbindung der Formel II solche der Fluor-, Chlor- oder Bromwasserstoffsäure, der Schwefel- oder Phosphorsäure verwendet.

8. Verfahren gemäß einem oder mehreren der Ansprüche 1 bis 7, dadurch gekennzeichnet, daß man bei Reaktionstemperaturen von -20 °C bis 200 °C arbeitet.

9. Verfahren gemäß einem oder mehreren der Ansprüche 1 bis 8, dadurch gekennzeichnet, daß man bei Reaktionstemperaturen von -10 °C bis 150 °C arbeitet.

**10.** Verfahren gemäß einem oder mehreren der Ansprüche 1 bis 9, dadurch gekennzeichnet, daß man die Verbindung der Formel II oder deren Salz zu einer Lösung gibt, die mindestens 1 Äquivalent Base und mindestens 1 Mol Cyanamid an Verbindung der Formel II enthält, und die Umsetzung durchführt.

Europäisches Patentamt

# EUROPÄISCHER RECHERCHENBERICHT

Nummer der Anmeldung

**EP 91 11 5630**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 271 834 (HOECHST)<br>* Seiten 1-3; Ansprüche 1-8 * | 1,2,4-10 | C 07 D 239/52<br>C 07 D 239/58 |
| D,X | EP-A-0 024 200 (DU PONT)<br>* Seiten 1-7,12 * | 1-8 | |
| P,A | EP-A-0 424 849 (HOECHST)<br>* Seiten 1-5 * | 1,4-10 | |

RECHERCHIERTE SACHGEBIETE (Int. Cl.5)

C 07 D 239/00

Der vorliegende Recherchenbericht wurde für alle Patentansprüche erstellt

| Recherchenort | Abschlußdatum der Recherche | Prüfer |
|---|---|---|
| Den Haag | 31 Oktober 91 | FRANCOIS J.C.L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument